Europäisches Patentamt

European Patent Office

Office européen des brevets

(11) Numéro de publication : **0 370 850 B1**

(12) **FASCICULE DE BREVET EUROPEEN**

(19)

(45) Date de publication du fascicule du brevet :
04.08.93 Bulletin 93/31

(21) Numéro de dépôt : **89403013.9**

(22) Date de dépôt : **02.11.89**

(51) Int. Cl.⁵ : **A01N 59/00**

(54) **Agent d'hygiène en hémodialyse.**

Le dossier contient des informations techniques présentées postérieurement au dépôt de la demande et ne figurant pas dans le présent fascicule.

(30) Priorité : **23.11.88 FR 8815252**

(43) Date de publication de la demande :
**30.05.90 Bulletin 90/22**

(45) Mention de la délivrance du brevet :
**04.08.93 Bulletin 93/31**

(84) Etats contractants désignés :
**AT BE CH DE ES FR GB GR IT LI LU NL SE**

(56) Documents cités :
**EP-A- 0 193 416**
**DE-A- 2 623 917**
**DE-A- 3 134 050**
**FR-A- 2 584 503**

(73) Titulaire : **L'AIR LIQUIDE, SOCIETE ANONYME POUR L'ETUDE ET L'EXPLOITATION DES PROCEDES GEORGES CLAUDE**
**75, Quai d'Orsay**
**F-75321 Paris Cédex 07 (FR)**

(72) Inventeur : **Nicolle, Rémy**
**12, Rue Martin Bernard**
**F-75013 Paris (FR)**
Inventeur : **Jourdan-Laforte, Eric**
**91, Rue Michel-Ange**
**F-75016 Paris (FR)**

(74) Mandataire : **Le Moenner, Gabriel et al**
**L'AIR LIQUIDE 75, Quai d'Orsay**
**F-75321 Paris Cédex 07 (FR)**

## Description

La présente invention concerne un agent d'hygiène en hémodialyse.

Le principe de la dialyse extra corporelle repose sur la mise en contact du sang à épurer avec un dialysat au travers d'une membrane semi-perméable. Pour que des échanges efficaces puissent se faire, il faut renouveler en permanence le sang et le dialysat, car la vitesse de diffusion, ou de dialyse, est fonction de la différence des concentrations entre le sang et le dialysat. Ceci est réalisé en créant une circulation extra-corporelle.

Toute chaîne de dialyse est constituée par un générateur, un dialyseur, un dialysat et un circuit d'eau.

Les générateurs permettent la fabrication du dialysat, la circulation du sang et du dialysat, et les contrôles des paramètres de dialyse.

Le rein artificiel ou dialyseur permet l'élimination de l'eau et des déchets produits par l'organisme en mettant en contact le sang avec un liquide, le dialysat, ou bain de dialyse ; sang et dialysat n'étant seulement séparés l'un de l'autre que par une mince membrane artificielle organique, de cellophane, cuprophane, polysulfone, polypropylène, polyamide, polyacétate, copolymère d'acrylonitrile... Cette membrane peut être disposée sous la forme de plaques, compartiments empilés les uns sur les autres, ou sous forme de bobines, compartiment unique enroulé en spirale autour d'un axe, ou enfin de capillaires, ensemble de très fines fibres creuses.

Ainsi, il existe trois types de dialyseurs, souvent livrés stériles et à usage unique.

Dans les reins bobines, du type à enroulement parallèle de deux feuillets de membrane dialysante en spirale autour d'un axe, le sang circule à l'intérieur de l'enveloppe formée par les deux feuillets de la membrane et le dialysat à l'extérieur. Le rein bobine est actuellement le moins utilisé.

Les reins plaques sont tous disposés selon le même schéma correspondant à un ensemble de 15 à 20 feuillets de membrane assemblés en plaques comme un sandwich, de telle sorte que compartiments de sang et compartiments de dialysat sont intercalés. Le sang circule de haut en bas entre les feuillets, le dialysat circule en sens inverse.

Le rein à fibres creuses contient très peu de sang ; il oppose une résistance très faible au passage du sang. A la place des feuillets de membrane en rouleaux ou en plaques, la surface dialysante est ici formée par des milliers de fibres creuses minuscules. Le sang les traverse de haut en bas et le dialysat passe autour dans l'autre sens.

Le dialysat est un mélange d'eau et d'électrolytes, la concentration des électrolytes dans le dialysat est établie de telle façon que la composition du sang en électrolytes soit normale en fin de séance.

L'eau pour les services d'hémodialyse doit être très pure, les chaînes d'hémodialyse sont en général équipées d'un traitement d'eau constitué d'adoucisseurs, de diminéralisation, d'osmoseur, de filtres, et d'un bac de stockage d'eau. Après le bac de stockage d'eau on trouve le circuit d'utilisation qui alimente les générateurs de dialyse.

Depuis l'apparition des techniques d'épuration sanguine extra-corporelle, des problèmes liés à la désinfection et au détartrage du matériel utilisé se sont toujours posés.

Les dialyseurs sont souvent des matériels considérés à usage unique. Néanmoins certains établissements les réutilisent sous la responsabilité des médecins, or la chaîne de dialyse peut se contaminer très facilement, c'est pour cela qu'il faut impérativement la désinfecter régulièrement.

Des rapports cliniques indiquent que des proliférations microbiennes importantes dans les appareils d'hémodialyse peuvent avoir des effets graves sur les patients dialysés. Il a été observé plus particulièrement des phénomènes de bactériémie, d'hépatite, de fièvre, de frissons, d'hypotension et de choc.

La stérilisation à chaud qui représenterait la mesure d'hygiène idéale est actuellement irréalisable à cause de nombreux éléments peu stables à la chaleur de la chaîne de dialyse.

L'adjonction de filtres étanches placés immédiatement avant le dialyseur ne donne pas toute satisfaction, car ces filtres peuvent être traversés par des virus, des mycoplasmes et certaines bactéries.

Même le recours à ces méthodes physiques de désinfection demande en plus une désinfection chimique régulière des autres éléments de la chaîne de dialyse.

Si l'on tient compte de toutes les exigences auxquelles un désinfectant approprié doit satisfaire, à savoir : un large spectre d'activité pour un temps d'action le plus court possible, des propriétés de nettoyage et de préservation du matériel ainsi que la capacité d'être facilement rincé dans des conditions contrôlées, seuls quelques composés peuvent être envisagés.

Les produits utilisés pour la désinfection sont généralement le formol ou l'hypochlorite de sodium.

Le circuit d'eau peut être désinfecté régulièrement au formol (aldéhyde formique) ou à l'hypochlorite de sodium, cette opération est réalisée en général une fois tous les six mois car elle est longue et nécessite un arrêt du service d'hémodialyse.

L'hypochlorite de sodium est utilisé à des dilutions plus ou moins importantes selon l'emploi ; pour la désinfection des générateurs de l'ordre de 50 %.

L'aldéhyde formique est également utilisé à des dilutions plus ou moins importantes selon l'emploi.

Ces produits de désinfection exigent des manipulations multiples, telles que préparations et dilutions... et présentent certains risques. Ainsi, l'aldéhyde formique est allergisant et son intolérance bien connue classée comme maladie professionnelle. Et l'hypochlorite de sodium apporte du chlore dans un milieu très pur, ce qui représente un inconvénient.

Des compositions aseptisantes pour le nettoyage et la stérilisation des lentilles de contact, qui contiennent 1-8% en poids de peroxyde d'hydrogène, 0.005-0.1% en poids d'acide peracétique et d'un quantité d'acide acétique nécessaire pour atteindre l'équilibre du système, ont été décrites dans le document EP- 193 416.

Indépendamment de la désinfection, des acides divers sont employés pour le détartrage, tels que les acides acétique, lactique, citrique...

L'acide peracétique, dont les propriétés désinfectantes sont connues, a été proposé à une concentration de plusieurs pourcents, de l'ordre de 3,5 - 4 %, dans la désinfection des générateurs de dialyse. A cette concentration, ce type de désinfectant présente divers inconvénients dont celui d'un produit présentant des risques pour le personnel soignant en raison de son action irritante pour la peau et les muqueuses, ainsi qu'une agressivité corrosive vis-à-vis du matériel de dialyse.

Aussi, il a été recherché un nouveau concept d'agent d'hygiène en hémodialyse pour satisfaire aux conditions suivantes : de non toxicité et biocompatibilité à l'état de traces, de désinfection et détartrage conjugués, de courte durée d'action inférieure à 30 minutes, de désinfectant à spectre complet, facilement rinçable et détectable, biodégradable, stable et prêt à l'emploi.

Il a été trouvé un agent d'hygiène en hémodialyse remplissant toutes ces conditions. En outre, cette formulation originale à base d'acide peracétique exalte les qualités d'un de ses principes actifs. Elle est constituée par une solution aqueuse contenant du peroxyde d'hydrogène à une concentration pondérale de 6 à 8 %, de l'acide peracétique à une concentration pondérale de 0,3 à 1 % et de l'acide acétique à une concentration pondérale de 2 à 10 %.

Une solution aqueuse contenant 6 à 8 % de peroxyde d'hydrogène, 0,3 à 0,4 % d'acide peracétique et 3,5 à 4,5 % d'acide acétique est très avantageuse, et en particulier une solution aqueuse contenant 7 % de peroxyde d'hydrogène, 0,35 % d'acide peracétique et de 3,5 à 4 % d'acide acétique.

Ces solutions aqueuses constituant un agent d'hygiène en hémodialyse se présentent sous la forme de liquide incolore, ininflammable, miscible à l'eau en toute proportion, ne moussant pas, se rinçant facilement, se conservant à la température ambiante dans son emballage d'origine, d'excellente stabilité pendant au moins une année.

Cet agent d'hygiène en hémodialyse constitue un produit inoffensif dans les conditions normales d'utilisation, tout en permettant la désinfection et le détartrage du matériel d'hémodialyse en une seule opération, dans les meilleures conditions d'hygiène et de sécurité pour le personnel et les patients, il n'a été observé ni irritations, ni allergies.

Avec une formule désinfectante à spectre complet, le nouvel agent d'hygiène en hémodialyse a fait la preuve de son efficacité bactéricide, fongicide, sporicide et virucide. Oxydant et acide, l'agent d'hygiène en hémodialyse libère un oxygène particulièrement actif. Il réagit avec les protéines de la paroi des micro-organismes et pénètre à l'intérieur des cellules pour les détruire. Et, son caractère acide lui donne des propriétés détartrantes.

L'activité bactéricide de l'agent d'hygiène en hémodialyse a été testée sur du matériel d'hémodialyse séquentiellement contaminé par les germes suivants : Pseudomonas aeruginosa et Mycobacterium smegmatis. Selon les dilutions effectuées par les appareils, 3 à 10 % de solution aqueuse à 7 % $H_2O_2$, 0,35 % d'acide peracétique et 3,5 % d'acide acétique, permettent d'obtenir une destruction de $10^6$ à $10^{10}$ bactéries/ml en 10 à 30 minutes.

L'agent d'hygiène en hémodialyse a également été testé in-vitro selon les protocoles des normes AFNOR, et il est important de noter que les propriétés désinfectantes sont conservées en présence de matières organiques (dialysat, sang, etc...).

L'activité fongicide de l'agent d'hygiène a été mise en évidence in-vitro contre les levures et les moisissures.

L'activité sporicide a été testée in-vitro et également sur du matériel d'hémodialyse contaminé par des spores de Bacillus cereus. Selon les appareils utilisés, 3 à 10 % d'une solution d'agent hygiénique à 0,35 % d'acide peracétique détruisent $10^4$ à $10^6$ spores de Bacillus cereus en 10 à 30 minutes.

L'activité virucide a été confirmée, notamment sur le virus du Sida. Un contact de 30 minutes avec la solution à 0,35 % d'acide peracétique, à la concentration de 1,5 % suffit à inactiver le HIV (Human Immunodeficiency Virus).

L'agent d'hygiène selon l'invention n'est pas toxique pour les patients et pour le personnel. Au contraire, on constate la disparition des chocs allergiques et anaphylactiques, ainsi que la diminution des réactions pyrogènes, souvent rencontrés au cours des dialyses.

La dose léthale DL 50 sur le rat est de 1.540 mg/kg pour l'acide peracétique, ce qui correspond à environ 440 g/kg de solution d'agent d'hygiène à 0,35 % d'acide peracétique. La toxicité par inhalation aigue : LC 50 est d'environ 150 g/m$^3$ de la dite solution, la toxicité subaigue correspond à 20 g/m$^3$ et le seuil tolérable pour le nez et les yeux est de 0,3 g/m$^3$. La toxicité cutanée, quant aux lésions réversibles de la muqueuse occulaire correspond à la solution pure, le seuil d'irritation de l'oeil correspond à la solution diluée au dixième. Le pouvoir irritant sur la peau est léger avec la solution pure et nul avec la solution diluée au dixième.

L'agent d'hygiène en hémodialyse est biocompatible, il est métabolisable à l'état de traces. Après 10.000 dialyses, aucune intolérance, irritation ou allergie n'ont été observées chez les patients et le personnel soignant. Des essais effectués sur du sang de donneurs sains ont montré que des doses inférieures à 0,2 de solution d'agent d'hygiène à 0,35 % en acide peracétique sont inoffensives et ne provoquent aucune modification des hématies (numérations inchangées), hémoglobine, plaquettes, leucocytes et protéines plasmatiques. Ainsi, une concentration 150 fois supérieure au seuil de détection des tests de contrôle de rinçage ne provoque aucun effet néfaste sur le sang humain in-vitro.

L'agent d'hygiène selon l'invention trouve des applications dans la désinfection et le détartrage de tous les stades de la chaîne de dialyse.

Il permet de désinfecter et de détartrer les générateurs de dialyse en moins d'une heure, temps de rinçage compris. Il peut être utilisé pour la désinfection rapide entre deux séances de dialyse. Le détartrage des générateurs est réalisé en même temps que leur désinfection ; il est inutile d'employer un autre acide. La désinfection s'effectue en utilisant le cycle de désinfection chimique des générateurs, les temps de contact et de rinçage sont réglés sur les appareils, respectivement en général 20 à 30 minutes de contact et 30 minutes de rinçage à l'eau osmosée. Pour un cycle normal de désinfection chimique il est recommandé d'utiliser une dilution comprise entre $^1$/10 et $^1$/35ème, de préférence au $^1$/30ème.

L'application s'étend au maintien de la stérilité des générateurs en attente d'utilisation dans le chaîne de dyalise.

L'agent d'hygiène selon l'invention permet de désinfecter les circuits de traitement d'eau pour hémodialyse, en particulier de la boucle de recirculation. Cet agent désinfectant s'emploie très facilement avec des temps de contact courts, environ 30 minutes. Sa facilité d'emploi permet d'envisager des traitements hebdomadaires ou quotidiens selon les besoins. Il désinfecte canalisations, filtres et cuves avec une chute de 10$^6$ à 10$^7$ germes/ml. La désinfection est suivie d'un rinçage à l'eau osmosée, pendant un temps court de l'ordre de 30 minutes à 2 heures selon les circuits.

La nouvelle composition d'acide peracétique - peroxyde d'hydrogène - acide acétique, est utilisable comme seul agent de nettoyage et de désinfection dans la stérilisation des dialyseurs, hémofiltres, hémodialyseurs. La réutilisation des dialyseurs traités avec la dite composition constitue une méthode simple très efficace, bien acceptée par le personnel soignant. Elle assure un nombre moyen de réutilisation de 8 à 12 fois par dialyseur, dans des conditions de haute sécurité bactériologique permettant ainsi d'envisager une réduction significative du coût global de la thérapeutique tout en permettant l'utilisation de membranes "hautement perméables" (de meilleure qualité mais très chères).

L'action germicide de l'agent de désinfection se prolonge pendant au moins 15 jours donnant ainsi la possibilité d'une conservation facile des modules de dialyse d'une séance à l'autre.

Dans les conditions de réutilisation automatisée, la dilution optimale pour décaper au mieux les dialyseurs semble se situer entre le 1/10ème et le 1/3,5ème. Dans ce cas, le nombre moyen de réutilisation est de 8 à 9 avec un seul reconditionnement des modules de dialyse de façon automatique à l'aide d'un appareil de réutilisation assurant un rinçage et un nettoyage du compartiment sanguin à haut débit dans le sens des fibres ainsi que par rétrofiltration. La réutilisation des dialyseurs avec les dilutions recommandées ne s'est jamais accompagnée de dégradation des filtres ou du matériel de réutilisation.

Il est donné ci-après des exemples non limitatifs d'application de l'agent d'hygiène selon l'invention désigné par Dialox®.

## EXEMPLE 1

### Désinfection des générateurs de dialyse.

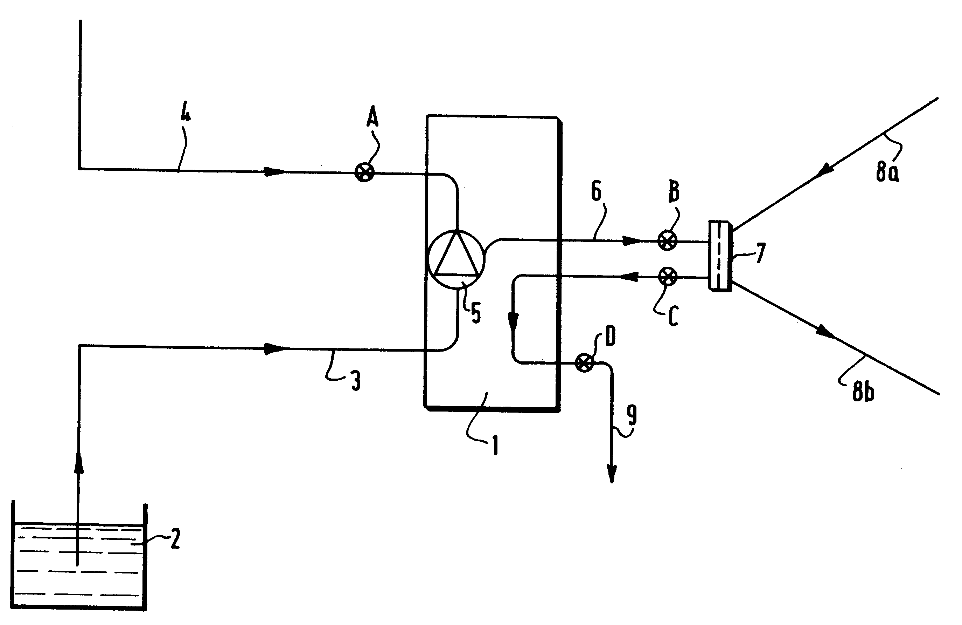

La chaîne de dialyse dans laquelle les essais de désinfection ont été réalisés, est représentée sur la figure annexée. Cette chaîne comprend le générateur (1) dans lequel s'effectue la préparation du bain de dialyse, à partir du dialysat prélevé dans le réservoir (2) et véhiculé vers le générateur par le circuit (3), l'eau circule dans le circuit (4) jusqu'au générateur, la dilution du bain de dialyse est réalisée par l'intermédiaire de la pome doseuse (5). Le bain de dialyse quitte le générateur par le circuit (6) pour être introduit dans le dialyseur (7), après échange avec le sang du malade selon le circuit (8a) (8b), le bain de dialyse est rejeté à l'égout par le circuit (9).

Pour cette série d'essais le générateur est mis en position de désinfection, et les essais ont été conduits avec diverses souches de germes volontairement introduites sur le circuit (3), le générateur prélevant sur le circuit (3) et diluant automatiquement l'agent d'hygiène Dialox® par l'action de la pompe doseuse (5).

Ces essais, ont été conduits dans divers types de générateur, avec des variations de dilutions de Dialox® correspondant à une solution contenant 0,35 % d'acide peracitique, 7 % $H_2O_2$ et 3,5 % d'acide acétique et des temps de contact de 10 à 30 minutes.

Les prélèvements ont été effectués respectivement aux points (A) sur le circuit d'eau (4) avant entrée dans le générateur, (B) sur le circuit (6) du bain de dialyse avant entrée dans le dialyseur, (C) sur le circuit du bain de dialyse (9) près de la sortie du dialyseur, et en (D) avant rejet à l'égoût.

L'introduction des germes est contrôlée, ainsi que les prélèvement effectués par une série de sondes, en entrée de l'eau osmosée (A), entrée du rein (B), sortie du rein (C) et sortie égoût (D). Dans chaque cas, il est donné la numération initiale et finale et la chute de germes.

EXEMPLE 1a

Générateur type Frésenius®.

TABLEAU Ia

| : Souche | : Pseudomonas aeruginosa | : |
| : Dilution | : DIALOX® 1/35ème | : |
| : T. de contact | : 20 mn | : |

| : Lieu de<br>: Prélèvement | : | Numération<br>initiale<br>/ml | : | Numération<br>finale<br>/50ml | : | Chute<br>log 10 | : |
|---|---|---|---|---|---|---|---|
| : A | : | 0 | : | 0 | : | 0,00 | : |
| : B | : | 8,00E+08 | : | 0 | : | 10,60 | : |
| : C | : | 8,00E+08 | : | 0 | : | 10,60 | : |
| : D | : | 8,00E+08 | : | 0 | : | 10,60 | : |

| : Souche | : Spores de Bacillus cereus | : |
| : Dilution | : DIALOX® 1/35ème | : |
| : T. de contact | : 20 mn | : |

| : Lieu de<br>: Prélèvement | : | Numération<br>initiale<br>/ml | : | Numération<br>finale<br>/50ml | : | Chute<br>log 10 | : |
|---|---|---|---|---|---|---|---|
| : A | : | 0 | : | 0 | : | 0,00 | : |
| : B | : | 3,00E+06 | : | 0 | : | 8,18 | : |
| : C | : | 3,00E+06 | : | 0 | : | 8,18 | : |
| : D | : | 6,00E+06 | : | 0 | : | 8,48 | : |

EXEMPLE 1b

Générateur type Monitral (HOSPAL)®

TABLEAU Ib

| Souche | : Pseudomonas aeruginosa |
|---|---|
| Dilution | : DIALOX® 1/35ème |
| T. de contact | : 30 mn |

| Lieu de Prélèvement | Numération initiale /ml | Numération finale /50ml | Chute log 10 |
|---|---|---|---|
| A | 0 | 0 | 0,00 |
| B | 1,00E+06 | 0 | 7,70 |
| C | 1,00E+06 | 0 | 7,70 |
| D | 1,00E+07 | 0 | 8,70 |

| Souche | : Mycobacterium smegmatis |
|---|---|
| Dilution | : DIALOX® 1/35ème |
| T. de contact | : 30 mn |

| Lieu de Prélèvement | Numération initiale /ml | Numération finale /50ml | Chute log 10 |
|---|---|---|---|
| A | 0 | 0 | 0,00 |
| B | 1,00E+06 | 0 | 7,70 |
| C | 1,00E+06 | 0 | 7,70 |
| D | 1,00E+07 | 0 | 8,70 |

| Souche | : Spores de Bacillus cereus |
|---|---|
| Dilution | : DIALOX® 1/35ème |
| T. de contact | : 30 mn |

| Lieu de Prélèvement | Numération initiale /ml | Numération finale /50ml | Chute log 10 |
|---|---|---|---|
| A | 0 | 0 | 0,00 |
| B | 4,00E+06 | 0 | 8,30 |
| C | 4,00E+06 | 0 | 8,30 |
| D | 7,00E+06 | 0 | 8,54 |

EXEMPLE 1c

Générateur type AK 10 (GAMBRO)®

TABLEAU Ic

| Souche | : Pseudomonas aeruginosa |
| Dilution | : DIALOX® 1/35ème |
| T. de contact | : 20 mn |

| Lieu de Prélèvement | Numération initiale /ml | Numération finale /50ml | Chute log 10 |
|---|---|---|---|
| A | 0 | 0 | 0,00 |
| B | 6,00E+08 | 0 | 10,48 |
| C | 6,00E+08 | 0 | 10,48 |
| D | 6,00E+08 | 0 | 10,48 |

| Souche | : Mycobacterium smegmatis |
| Dilution | : DIALOX® 1/35ème |
| T. de contact | : 10 mn |

| Lieu de Prélèvement | Numération initiale /ml | Numération finale /50ml | Chute log 10 |
|---|---|---|---|
| A | 0 | 0 | 0,00 |
| B | 1,00E+07 | 0 | 8,70 |
| C | 1,00E+07 | 0 | 8,70 |
| D | 9,00E+07 | 0 | 9,65 |

| Souche | : Spores de Bacillus cereus |
| Dilution | : DIALOX® 1/35ème |
| T. de contact | : 10 mn |

| Lieu de Prélèvement | Numération initiale /ml | Numération finale /50ml | Chute log 10 |
|---|---|---|---|
| A | 0 | 0 | 0,00 |
| B | 1,00E+06 | 900 | 4,74 |
| C | 1,00E+06 | 700 | 4,85 |
| D | 2,00E+06 | 1000 | 5,00 |

| Souche | Spores de Bacillus cereus |
|---|---|
| Dilution | DIALOX ® 1/35ème |
| T. de contact | 10 mn |

| Lieu de Prélèvement | Numération initiale /ml | Numération finale /50ml | Chute log 10 |
|---|---|---|---|
| A | 0 | 0 | 0,00 |
| B | 8,00E+06 | 5000 | 4,90 |
| C | 8,00E+06 | 4000 | 5,00 |
| D | 1,00E+07 | 3000 | 5,22 |

| Souche | Spores de Bacillus cereus |
|---|---|
| Dilution | DIALOX ® 1/35ème |
| T. de contact | 10 mn |

| Lieu de Prélèvement | Numération initiale /ml | Numération finale /50ml | Chûte log 10 |
|---|---|---|---|
| A | 0 | 0 | 0,00 |
| B | 4,00E+06 | 0 | 8,30 |
| C | 4,00E+06 | 0 | 8,30 |
| D | 4,00E+06 | 0 | 8,30 |

EXEMPLE 1d

Essai comparatif de désinfection d'un générateur type AK 10 (GAMBRO) ®

TABLEAU Id

| Souche | Spores de Bacillus cereus |
|---|---|
| Dilution | Formol 4 % |
| T. de contact | 12 heures |

| Lieu de Prélèvement | Numération initiale /ml | Numération finale /50ml | Chûte log 10 |
|---|---|---|---|
| A | 0 | 0 | 0,00 |
| B | 1,00E+06 | 300 | 5,22 |
| C | 1,00E+06 | 500 | 5,00 |
| D | 1,00E+06 | 150 | 5,52 |

<u>EXEMPLE 2</u>

Etude ex-vivo du pouvoir germicide d'une solution aqueuse à 3,5 % d'acide peracétique, 7 % de $H_2O_2$ et 3,5 % d'acide acétique (Dialox ®).

Le pouvoir germicide de la solution de Dialox® a été étudié in vitro sur des hémodiafiltres utilisés (HF 80, membrane polysulfone, Frésénius ® ) contaminés séquentiellement par 3 types de germe (Pseudomonas aeruginosa, Mycobacterium smegmatis et Bacillus cereus sous forme sporulée.

Pour chaque type de germe 5 hémodiafiltres furent utilisés : le premier servant de témoin de contamination et les 4 autres servant de témoins d'efficacité du produit désinfectant. Ces hémodiafiltres furent sousmis à la procédure habituelle de réutilisation avec désinfection par passage au Renatron ®, appareil de réutilisation assurant un rinçage et un nettoyage du compartiment sanguin à haut débit dans le sens de fibres ainsi que par rétrofiltration.

Ces temps de contact des membranes avec la solution désinfectante furent respectivement de 5, 10, 20 et 30 minutes, et le dénombrement des colonies de germes recueillies sur les membranes fut fait à la 24ème, 48ème, 72ème et 96ème heure de culture.

Le pouvoir bactéricide du Dialox ® exprimé en titre de réduction logarithmique du nombre de germes par ml obtenu pour les trois catégories de germes testés en fonction du temps est représenté sur le tableau suivant.

TABLEAU II

| TEMPS MINUTES | NUMEROTATION A T=0 | NUMEROTATION APRES TEMPS DE CONTACT | Δ REDUCTION LOG 10 |
|---|---|---|---|
| PSEUDOMONAS AEROGINOSA | | | |
| 5 | $1,2. \ 10^7$ g/ml | 0 | 7,08 |
| 10 | $1,2. \ 10^7$ g/ml | 0 | 7,08 |
| 15 | $1,2. \ 10^7$ g/ml | 0 | 7,08 |
| 20 | $1,2. \ 10^7$ g/ml | 0 | 7,08 |
| MYCOBATERIUM SMEGMATIS | | | |
| 5 | $5,1. \ 10^7$ g/ml | 0 | 7,71 |
| 10 | $5,1. \ 10^7$ g/ml | 0 | 7,71 |
| 15 | $5,1. \ 10^7$ g/ml | 0 | 7,71 |
| 20 | $5,1. \ 10^7$ g/ml | 0 | 7,71 |
| BACILLUS CEREUX SPORULE | | | |
| 5 | $4,0. \ 10^7$ g/ml | 0 | 6,60 |
| 10 | $4,0. \ 10^7$ g/ml | 0 | 6,60 |
| 15 | $4,0. \ 10^7$ g/ml | 0 | 6,60 |
| 20 | $4,0. \ 10^7$ g/ml | 0 | 6,60 |

Pour une solution contaminante comportant $10^6$ à $10^7$ unités formant colonies (U.F.C.)/ml, le titre de réduction tant sur le secteur sanguin que sur le secteur dialysat est de 6 à 7 log après 5 minutes, de contact pour une solution de Dialox® au $1/10$ème. Le titre de réduction est identique pour les germes sporulés ou non. L'effet bactéricide paraît ainsi absolu pour une dilution de Dialox ® au $1/10$ème après 5 minutes de contact.

<u>EXEMPLE 3</u>

Etude in-vivo de l'effet nettoyant et désinfectant du Dialox® sur les modules de dialyse.

Cette évaluation in-vivo a été faite sur dix patients insuffisants rénaux chroniques stables, en traitement de suppléance extrarénale, par hémodialyse, hémofiltration ou hémodiafiltration.

Trois modèles de dialyseurs ont été utilisés : HF 80 (membrane polysulfone Frésénius ®), Filtral 16 membrane polyacrylonitrile AN69 HOSPAL ®, FH 88 (membrane polyamide, GAMBRO ® ). 6 dialyseurs ont été utilisés pour chaque patient.

Le reconditionnement des modules de dialyse fut réalisé de façon automatique sur un Renatron ® dès la fin de séance.

L'agent de nettoyage fut le Dialox ® à 0,35 % d'acide peracétique. Pour le nettoyage des filtres, des dilutions au 1/3,5ème, 1/7ème, 1/10ème, 1/20ème furent utilisées. La dilution employée pour le maintien de stérilité des filtres d'une séance à l'autre fut de 1/10ème.

Le tableau III indique le nombre moyen de réutilisation de 60 modules chez 10 patients (représentés par leurs initiales) à 2 dilutions de Dialox ® à 0,35 % d'acide peracétique, respectivement 1/20ème et 1/3,5ème.

## TABLEAU III

### Influence du patient

| Patient | BC | CJ | RY | FA | EK |
|---|---|---|---|---|---|
| Filtre | FH88 | FH88 | FH88 | HF80 | HF80 |
| Dilution de Dialox ® | | | | | |
| 1/20ème X | 7 | 8,2 | 5,3 | 4,3 | 9,5 |
| 1/3,5ème X | 8,5 | 7,7 | 6,2 | 6,0 | 9,8 |

| Patient | EA | EF | TR | DV | FP |
|---|---|---|---|---|---|
| Filtre | HF80 | HF80 | HF80 | F16 | F16 |
| Dilution de Dialox ® | | | | | |
| 1/20ème X | 9,7 | 9,8 | 9,2 | 7,7 | 11,2 |
| 1/3,5ème X | 10 | 10,2 | 9,8 | 7,8 | 10,3 |

X : nombre de réutilisation moyen/patient.

Le nombre moyen de réutilisation fut ainsi de 8 (extrêmes 4 à 11 fois) avec une bonne reproductibilité chez un même patient et ce quelle que soit la dilution de Dialox® utilisée.

Sur le tableau IV a été représenté le nombre moyen de réutilisation des modules de dialyse selon leur mode d'utilisation en hémodialyse (HD), en hémofiltration (HF) ou en hémodiafiltration (HDF) à 4 dilutions différentes de Dialox ® à 0,35 % d'acide peracétique, respectivement $^1$/20ème, $^1$/10ème, $^1$/7ème et $^1$/3,5ème.

<div align="center">

TABLEAU IV

Influence de la méthode de dialyse

</div>

| Dilution de DIALOX ® | HF | HDF | HD |
|---|---|---|---|
| 1/20ème | | | |
| X | 6,2 | 9,5 | 9,4 |
| n | (18) | (30) | (12) |
| 1/10ème | | | |
| X | 7,5 | 9 | 10,5 |
| n | (3) | (5) | (2) |
| 1/7ème | | | |
| X | 7,1 | 10,0 | 9,1 |
| n | (18) | (30) | (12) |
| 1/3,5ème | | | |
| X | 7,3 | 9,2 | 10,0 |
| n | (3) | (5) | (2) |

n : nombre total d'essais.
X : nombre de réutilisation moyen/methode.

L'influence de la méthode de dialyse est nette si l'on compare les 2 groupes les plus représentatifs, à savoir ceux correspondant à des dilutions au 1/20ème et 1/7ème. Ces données démontrent que les modules utilisés en hémofiltration ont un nombre de réutilisation moyen significativement plus bas que ceux utilisés en hémodialyse ou en hémodiafiltration.

L'influence du type de dialyseur a donné lieu à une étude pour les modules de dialyse FH 88, HF 80 et FILTRAL 16 à 4 dilutions différentes de Dialox ® 0,35 % d'acide peracétique, respectivement 1/20ème, 1/10ème, 1/7ème, 1/3,5ème.

Le nombre moyen de réutilisation (N) par dialyseur est consigné dans le tableau V.

## TABLEAU V

### Influence du type de dialyseur

| Dilution de DIALOX ® | FH88 | HF80 | F16 |
|---|---|---|---|
| 1/20ème | | | |
| X | 5,2 | 7,1 | 6,3 |
| n | (30) | (50) | (20) |
| 1/10ème | | | |
| X | 5,3 | 7,6 | 7,1 |
| n | (3) | (5) | (2) |
| 1/7ème | | | |
| X | 5,7 | 7,7 | 6,2 |
| n | (30) | (50) | (20) |
| 1/3,5ème | | | |
| X | 5,3 | 7,8 | 7,0 |
| n | (3) | (5) | (2) |

n : nombre total d'essais.
N : nombre de réutilisation moyen/dialyseur.

Ces données montrent qu'il existe une différence significative seulement entre les hémofiltres FH 88 ® et le groupe des hémodiafiltres HF 80 ® et FILTRAL 16 ®.

Au cours de ce programme de réutilisation des modules de dialyse aucune réaction particulière n'a été observée chez les patients, et les performances des dialyseurs ne furent pas affectées de façon significative par la réutilisation.

## EXEMPLE 4

Désinfection du circuit d'eau.

Dans ce type de désinfection, on utilise l'agent désinfectant à 0,35 % d'acide peracétique, Dialox ®, à la dose de 5 litres pour 100 litres d'eau est injecté dans une capacité tampon, pendant la désinfection, les pompes de circulation du circuit doivent être en marche pour permettre ainsi une circulation du mélange eau - agent désinfectant. Le temps de contact de la circulation/désinfection est de 30 minutes. Le rinçage du circuit se fait par de l'eau osmosée.

## Revendications

1. Agent d'hygiène en hémodialyse à base d'acide peracétique, caractérisé en ce qu'il est constitué par une solution aqueuse contenant du peroxyde d'hydrogène à une concentration pondérale de 6 à 8 %, de l'acide peracétique à une concentration pondérale de 0,3 à 1 % et de l'acide acétique à une concentation pondérale de 2 à 10 %.

2. Agent d'hygiène en hémodialyse selon la revendication 1, caractérisé en ce que la solution aqueuse contient 6 à 8 % de peroxyde d'hydrogène, 0,3 à 0,4 % d'acide peracétique, 3,5 à 4,5 % d'acide acétique.

3. Agent d'hygiène en hémodialyse selon la revendication 1 ou 2, caractérisé en ce que la solution aqueuse contient 7 % de peroxyde d'hydrogène, 0,35 % d'acide peracétique et entre 3,5 et 4 % d'acide acétique.

4. Application en hémodialyse d'un agent d'hygiène constitué par une solution aqueuse contenant du peroxyde d'hydrogène à une concentration pondérale de 6 à 8 %, de l'acide peracétique à une concentration pondérale de 0,1 à 1 % et de l'acide acétique à une concentration pondérale de 2 à 10 %, ou de l'agent d'hygiène selon l'une quelconque des revendications 1 à 3, à la désinfection chimique et au détartrage de tous les stades de la chaîne de dialyse.

5. Application en hémodialyse d'un agent d'hygiène constitué par une solution aqueuse contenant du peroxyde d'hydrogène à une concentration pondérale de 6 à 8 %, de l'acide peracétique à une concentration pondérale de 0,1 à 1 % et de l'acide acétique à une concentration pondérale de 2 à 10 %, ou de l'agent d'hygiène selon l'une quelconque des revendications 1 à 3, à la désinfection chimique et au détartrage des générateurs de dialyse.

6. Application en hémodialyse d'un agent d'hygiène constitué par une solution aqueuse contenant du peroxyde d'hydrogène à une concentration pondérale de 6 à 8 %, de l'acide peracétique à une concentration pondérale de 0,1 à 1 % et de l'acide acétique à une concentration pondérale de 2 à 10 %, ou de l'agent d'hygiène selon l'une quelconque des revendications 1 à 3, au maintien de la stérilité des générateurs en attente d'utilisation dans la chaîne de dialyse.

7. Application en hémodialyse d'un agent d'hygiène constitué par une solution aqueuse contenant du peroxyde d'hydrogène à une concentration pondérale de 6 à 8 %, de l'acide peracétique à une concentration pondérale de 0,1 à 1 % et de l'acide acétique à une concentration pondérale de 2 à 10 %, ou de l'agent d'hygiène selon l'une quelconque des revendications 1 à 3, à la désinfection et au détartrage des circuits de traitement d'eau pour hémodialyse, en particulier de la boucle de recirculation.

8. Application en hémodialyse d'un agent d'hygiène constitué par une solution aqueuse contenant du peroxyde d'hydrogène à une concentration pondérale de 6 à 8 %, de l'acide peracétique à une concentration pondérale de 0,1 à 1 % et de l'acide acétique à une concentration pondérale de 2 à 10 %, ou de l'agent d'hygiène selon l'une quelconque des revendications 1 à 3, à la stérilisation des dialyseurs, hémofiltres, hémodialyseurs.

9. Application en hémodialyse d'un agent d'hygiène constitué par une solution aqueuse contenant du peroxyde d'hydrogène à une concentration pondérale de 6 à 8 %, de l'acide peracétique à une concentration pondérale de 0,1 à 1 % et de l'acide acétique à une concentration pondérale de 2 à 10 %, ou de l'agent d'hygiène selon l'une quelconque des revendications 1 à 3, au maintien de la stérilité des dialyseurs en attente d'utilisation.

10. Procédé de désinfection et de détartrage des générateurs de dialyse selon la revendication 5, caractérisé en ce que l'agent d'hygiène en hémodialyse est mis en oeuvre à une dilution entre 1/10ème et 1/35ème avec un temps de contact de 20 à 30 minutes suivi d'un temps de rinçage à l'eau osmosée de 20 à 30 minutes.

11. Procédé de désinfection des circuits de traitement d'eau pour hémodialyse selon la revendication 7, avec mise en oeuvre de l'agent d'hygiène en hémodialyse à raison de 2 à 10 litres pour 100 litres d'eau, suivie d'un rinçage à l'eau osmosée.

12. Procédé de stérilisation des dialyseurs, hémofiltres, hémodialyseurs selon la revendication 8, caractérisé en ce que dans les conditions de réutilisation automatisée, l'agent d'hygiène est mis en oeuvre à une dilution de 1/10ème à 1/3,5ème.

**Patentansprüche**

1. Hygienemittel für die Hämodialyse auf der Basis von Peressigsäure, dadurch gekennzeichnet, daß es aus einer wässrigen Lösung besteht, die Wasserstoffperoxid mit einer Gewichtskonzentration von 6 bis 8%, Peressigsäure mit einer Gewichtskonzentration von 0,3 bis 1% und Essigsäure mit einer Gewichtskonzentration von 2 bis 10% enthält.

2. Hygienemittel für die Hämodialyse, nach Anspruch 1, dadurch gekennzeichnet, daß die wässrige Lösung 6 bis 8% Wasserstoffperoxid, 0,3 bis 0,4% Peressigsäure und 3,5 bis 4,5% Essigsäure enthält.

3. Hygienemittel für die Hämodialyse, nach Anspruch 1 oder 2, dadurch gekennzeichnet, daß die wässrige Lösung 7% Wasserstoffperoxid, 0,35% Peressigsäure und 3,5 bis 4% Essigsäure enthält.

4. Verwendung eines Hygienemittels für die Hämodialyse, das aus einer wässrigen Lösung besteht, die Wasserstoffperoxid mit einer Gewichtskonzentration von 6 bis 8%, Peressigsäure mit einer Gewichtskonzentration von 0,1 bis 1% und Essigsäure mit einer Gewichtskonzentration von 2 bis 10% enthält, oder des Hygienemittels nach einem der Ansprüche 1 bis 3 zur chemischen Desinfektion und zur Entkalkung aller Stationen der Dialysekette.

5. Verwendung eines Hygienemittels für die Hämodialyse, das aus einer wässrigen Lösung besteht, die Wasserstoffperoxid mit einer Gewichtskonzentration von 6 bis 8%, Peressigsäure mit einer Gewichtskonzentration von 0,1 bis 1% und Essigsäure mit einer Gewichtskonzentration von 2 bis 10% enthält, oder des Hygienemittels nach einem der Ansprüche 1 bis 3 zur chemischen Desinfektion und zur Entkalkung von Dialysegeneratoren.

6. Verwendung eines Hygienemittels für die Hämodialyse, das aus einer wässrigen Lösung besteht, die Wasserstoffperoxid mit einer Gewichtskonzentration von 6 bis 8%, Peressigsäure mit einer Gewichtskonzentration von 0,1 bis 1% und Essigsäure mit einer Gewichtskonzentration von 2 bis 10% enthält, oder des Hygienemittels nach einem der Ansprüche 1 bis 3 zur Erhaltung der Sterilität von in Bereitschaft zum Einsatz in der Dialysekette stehenden Generatoren.

7. Verwendung eines Hygienemittels für die Hämodialyse, das aus einer wässrigen Lösung besteht, die Wasserstoffperoxid mit einer Gewichtskonzentration von 6 bis 8%, Peressigsäure mit einer Gewichtskonzentration von 0,1 bis 1% und Essigsäure mit einer Gewichtskonzentration von 2 bis 10% enthält, oder des Hygienemittels nach einem der Ansprüche 1 bis 3 zur Desinfektion und zur Entkalkung der Kreisläufe zur Wasseraufbereitung für die Hämodialyse, insbesondere der Rezirkulationsschleife.

8. Verwendung eines Hygienemittels für die Hämodialyse, das aus einer wässrigen Lösung besteht, die Wasserstoffperoxid mit einer Gewichtskonzentration von 6 bis 8%, Peressigsäure mit einer Gewichtskonzentration von 0,1 bis 1% und Essigsäure mit einer Gewichtskonzentration von 2 bis 10% enthält, oder des Hygienemittels nach einem der Ansprüche 1 bis 3 zur Sterilisation von Dialysatoren, Hämofiltern und Hämodialysatoren.

9. Verwendung eines Hygienemittels für die Hämodialyse, das aus einer wässrigen Lösung besteht, die Wasserstoffperoxid mit einer Gewichtskonzentration von 6 bis 8%, Peressigsäure mit einer Gewichtskonzentration von 0,1 bis 1% und Essigsäure mit einer Gewichtskonzentration von 2 bis 10% enthält, oder des Hygienemittels nach einem der Ansprüche 1 bis 3 zum Erhalten der Sterilität von in Einsatzbereitschaft stehenden Dialysatoren.

10. Verfahren zur Desinfektion und zur Entkalkung von Dialysegeneratoren nach Anspruch 5, dadurch gekennzeichnet, daß das Hygienemittel für die Hämodialyse in einer Verdünnung von 1:10 bis 1:35 mit einer Kontaktzeit zwischen 20 und 30 Minuten, gefolgt von einer Spülzeit mit osmotisiertem Wasser zwischen 20 und 30 Minuten, eingesetzt wird.

11. Verfahren zur Desinfektion von Kreisläufen zur Wasseraufbereitung für die Hämodialyse nach Anspruch 7, bei dem das Hygienemittel für die Hämodialyse im Verhältnis von 2 bis 10 Litern auf 100 Liter Wasser, gefolgt von einer Spülung mit osmotisiertem Wasser, eingesetzt wird.

12. Verfahren zur Sterilisation von Dialysatoren, Hämofiltern und Hämodialysatoren nach Anspruch 8, dadurch gekennzeichnet, daß im Fall der automatischen Wiederverwendung das Hygienemittel in einer Verdünnung von 1:10 bis 1:3,5 verwendet wird.

## Claims

1. Haemodialysis hygiene agent based on peracetic acid, characterised in that it consists of an aqueous solution containing hydrogen peroxide in a concentration of 6 to 8% by weight, peracetic acid in a concentration of 0.3 to 1% by weight and acetic acid in a concentration of 2 to 10% by weight.

2. Haemodialysis hygiene agent according to claim 1, characterised in that the aqueous solution contains 6 to 8% hydrogen peroxide, 0.3 to 0.4% peracetic acid and 3.5 to 4.5% acetic acid.

3. Haemodialysis hygiene agent according to claim 1 or 2, characterised in that the aqueous solution contains 7% hydrogen peroxide, 0.35% peracetic acid and between 3.5 and 4% acetic acid.

4. Use in haemodialysis of a hygiene agent consisting of an aqueous solution containing hydrogen peroxide in a concentration of 6 to 8% by weight, peracetic acid in a concentration of 0.1 to 1% by weight, peracetic acid in a concentration of 2 to 10% by weight, or of the hygiene agent according to any of claims 1 to 3, for the chemical disinfection and descaling of all stages of the dialysis chain.

5. Use in haemodialysis of a hygiene agent consisting of an aqueous solution containing hydrogen peroxide in a concentration of 6 to 8% by weight, peracetic acid in a concentration of 0.1 to 1% by weight and acetic acid in a concentration of 2 to 10% by weight, or of the hygiene agent according to any of claims 1 to 3, for the chemical disinfection and descaling of dialysis generators.

6. Use in haemodialysis of a hygiene agent consisting of an aqueous solution containing hydrogen peroxide in a concentration of 6 to 8% by weight, peracetic acid in a concentration of 0.1 to 1% by weight and acetic acid in a concentration of 2 to 10% by weight, or of the hygiene agent according to any of claims 1 to 3, for maintaining the sterility of generators while awaiting use in the dialysis chain.

7. Use in haemodialysis of a hygiene agent consisting of an aqueous solution containing hydrogen peroxide in a concentration of 6 to 8% by weight, peracetic acid in a concentration of 0.1 to 1% by weight and acetic acid in a concentration of 2 to 10% by weight, or of the hygiene agent according to any of claims 1 to 3, for the disinfection and descaling of the water treatment circuits for haemodialysis, in particular the recirculation loop.

8. Use in haemodialysis of a hygiene agent consisting of an aqueous solution containing hydrogen peroxide in a concentration of 6 to 8% by weight, peracetic acid in a concentration of 0.1 to 1% by weight and acetic acid in a concentration of 2 to 10% by weight, or of the hygiene agent according to any of claims 1 to 3, for the sterilisation of dialysers, blood filters and haemodialysers.

9. Use in haemodialysis of a hygiene agent consisting of an aqueous solution containing hydrogen peroxide in a concentration of 6 to 8% by weight, peracetic acid in a concentration of 0.1 to 1% by weight and acetic acid in a concentration of 2 to 10% by weight, or of the hygiene agent according to any of claims 1 to 3, for maintaining the sterility of dialysers while awaiting use.

10. Method for the disinfection and descaling of dialysis generators according to claim 5, characterised in that the haemodialysis hygiene agent is used in a dilution between 1/10 and 1/35 with a contact time of 20 to 30 minutes followed by a rinsing time of 20 to 30 minutes with water subjected to osmosis.

11. Method for the disinfection of the water treatment circuits of haemodialysis according to claim 7, with use of the haemodialysis hygiene agent at the rate of 2 to 10 litres per 100 litres of water, followed followed by rinsing with water subjected to osmosis.

12. Method for the sterilisation of dialysers, blood filters and haemodialysers according to claim 8, characterised in that under conditions of automated reuse, the hygiene agent is used with a dilution of 1/10 to 1/3.5.